# EUROPEAN PATENT SPECIFICATION

(11) **EP 0 464 154 B1**
(45) Date of publication and mention of the grant of the patent: **25.01.1995**
(21) Application number: 90907749.7
(22) Date of filing: 09.04.1990
(51) Int. Cl.: C07B 37/02, C07C 2/66, C07C 15/02, C07C 37/14

(54) **ALKYLATION OF AROMATICS**
ALKYLIERUNG VON AROMATEN
ALKYLATION DE COMPOSITIONS AROMATIQUES

(30) Priority: 25.01.1990 US 469998; 25.01.1990 US 469999; 25.01.1990 US 470015
(43) Date of publication of application: 08.01.1992
(73) Proprietor: MOBIL OIL CORPORATION, New York New York 10017 (US)
(72) Inventor: KUSHNERICK, John, Douglas, Boothwyn, PA 19061 (US); LE, Quang, Ngoc, Cherry Hill, NJ 08002 (US); MARLER, David, Owen, Deptford, NJ 08096 (US); McWILLIANS, John, Paul, Woodbury, NJ 08096 (US); RUBIN, Mae, Koenig, Bala Cynwyd, PA 19004 (US); SHIM, Joosup, Wenonah, NJ 08090 (US); SMITH, Charles, Morris, Princeton, NJ 08540 (US); WONG, Stephen, Sui, Fai, Medford, NJ 08055 (US)
(74) Representative: Colmer, Stephen Gary
(86) International application number: US9002038
(87) International publication number: WO9111411

(56) References cited:
- US-A- 4 283 583
- US-A- 4 291 185
- US-A- 4 849 570
- US-A- 4 891 458
- US-A- 4 899 008
- US-A- 4 916 100
- No further relevant documents have been disclosed.

## Description

This invention relates to a process alkylating an aromatic compound employing a synthetic porous crystalline zeolite as an alkylation catalyst.

Zeolitic materials, both natural and synthetic, have been demonstrated in the past to have catalytic properties for various types of hydrocarbon conversion. Certain zeolitic materials are ordered, porous crystalline aluminosilicates having a definite crystalline structure as determined by X-ray diffraction, within which there are a large number of smaller cavities which may be interconnected by a number of still smaller channels or pores. These cavities and pores are uniform in size within a specific zeolitic material. Since the dimensions of these pores are such as to accept for adsorption molecules of certain dimensions while rejecting those of larger dimensions, these materials have come to be known as "molecular sieves" and are utilized in a variety of ways to take advantage of these properties. Such molecular sieves, both natural and synthetic, include a wide variety of positive ion-containing crystalline silicates. These silicates can be described as a rigid three-dimensional framework of SiO₄ and Periodic Table Group IIIA element oxide, e.g., AlO₄, in which the tetrahedra are cross-linked by the sharing of oxygen atoms whereby the ratio of the total Group IIIA element, e.g., aluminum, and silicon atoms to oxygen atoms is 1:2. The electrovalence of the tetrahedra containing the Group IIIA element, e.g., aluminum, is balanced by the inclusion in the crystal of a cation, e.g., an alkali metal or an alkaline earth metal cation. This can be expressed wherein the ratio of the Group IIA element, e.g., aluminum, to the number of various cations, such as Ca/2, Sr/2, Na, K or Li, is equal to unity. One type of cation may be exchanged either entirely or partially with another type of cation utilizing ion exchange techniques in a conventional manner.

Prior art techniques have resulted in the formation of a great variety of synthetic zeolites. Many of these zeolites have come to be designated by letter or other convenient symbols, as illustrated by zeolite Z (U.S. Patent No. 2,882,243), zeolite X (U.S. Patent No. 2,882,244), zeolite Y (U.S. Patent No. 3,130,007), zeolite ZK-5 (U.S. Patent No. 3,247,195), zeolite ZK-4 (U.S. Patent No. 3,314,752), zeolite ZSM-5 (U.S. Patent No. 3,702,886), zeolite ZSM-11 (U.S. Patent No. 3,709,979), zeolite ZSM-12 (U.S. Patent No. 3,832,449), zeolite ZSM-20 (U.S. Patent No. 3,972,983), zeolite ZSM-35 (U.S. Patent No. 4,016,245), and zeolite ZSM-23 (U.S. Patent No. 4,076,842).

The SiO₂/Al₂O₃ ratio of a given zeolite is often variable. For example, zeolite X can be synthesized with SiO₂/Al₂O₃ ratios of from 2 to 3; zeolite Y, from 3 to 6. In some zeolites, the upper limit of the SiO₂/Al₂O₃ ratio is unbounded. ZSM-5 is one such example wherein the SiO₂/Al₂O₃ ratio is at least 5 and up to the limits of present analytical measurement techniques. U.S. Patent No. 3,941,871 (Re. 29,948) discloses a porous crystalline silicate made from a reaction mixture containing no deliberately added alumina and exhibiting the X-ray diffraction pattern characteristic of ZSM-5. U.S. Patent Nos. 4,061,724, 4,073,865 and 4,104,294 describe crystalline silicates of varying alumina and metal content.

Alkylation is one of the most important and useful reactions of hydrocarbons. Lewis and Bronsted acids, including a variety of natural and synthetic zeolites, have been used as catalysts. Alkylation of aromatic hydrocarbon compounds employing certain crystalline zeolite catalysts is known in the art. For instance, U.S. Patent No. 3,251,897 describes liquid phase alkylation in the presence of crystalline aluminosilicates such as faujasite, heulandite, clinoptilolite, mordenite, dachiardite, zeolite X and zeolite Y.

U.S. Patent Nos. 3,631,120 and 3,641,177 describe liquid phase processes for alkylation of aromatic hydrocarbons with olefins in the presence of certain zeolites.

U.S. Patent Nos. 3,751,504 and 3,751,506 describe the vapor phase alkylation of aromatic hydrocarbons with olefins in the presence of a specified type of zeolite catalyst.

U.S. Patent Nos. 3,755,483 and 4,393,262 disclose the vapor phase reaction of propylene with benzene in the presence of zeolite ZSM-12, to produce isopropylbenzene.

U.S. Patent No. 4,469,908 discloses the alkylation of aromatic hydrocarbons with relatively short chain alkylating agents having from one to five carbon atoms employing ZSM-12 as alkylation catalyst.

U.S. Patent No. 4,283,573 describes a process for producing relatively long chain alkylphenols by alkylation of phenol with a long chain alkylating agnnt possessing one or more available alkyl groups of at least 5 carbon atoms in length employing as catalyst a zeolite such as cancrinite, gmelinite, mordenite, offretite or ZSM-12.

The present invention resides in a process for alkylating an aromatic compound comprising contacting the aromatic compound with at least one alkylating agent in the presence of an alkylation catalyst comprising a synthetic porous crystalline zeolite having an X-ray diffraction pattern including values substantially as set forth in Table I of the specification.

The term "aromatic" in reference to the alkylatable compounds which are useful herein is to be understood in accordance with its art-recognized scope which includes substituted and unsubstituted mono- and polynuclear compounds. Compounds of an aromatic character which possess a hetero atom are also useful provided they do not act as catalyst poisons under the reaction conditions selected.

Substituted aromatic compounds which can be alkylated herein must possess at least one hydrogen atom directly bonded to the aromatic nucleus. The aromatic rings can be substituted with one or more alkyl, aryl, alkaryl, alkoxy, aryloxy, hydroxy, cycloalkyl, halide, and/or other groups which do not interfere with the alkylation reaction. In one particular embodiment, the aromatic compound is a phenolic compound.

Suitable aromatic hydrocarbons include benzene, toluene, xylene, naphthalene, anthracene, naphthacene, perylene, coronene and phenanthrene.

Generally the alkyl groups which can be present as substituents on the aromatic compound contain from one to 22 carbon atoms and preferably from one to eight carbon atoms, and most preferably from one to four carbon atoms.

Suitable alkyl substituted aromatic compounds include toluene, xylene, isopropylbenzene, normal propylbenzene, alpha-methylnaphthalene, ethylbenzene, cumene, mesitylene, durene, p-cymene, butylbenzene, pseudocumene, o-diethylbenzene, m-diethylbenzene, p-diethylbenzene, isoamylbenzene, isohexylbenzene, pentaethylbenzene, pentamethylbenzene; 1,2,3,4- tetraethylbenzene; 1,2,3,5-tetramethylbenzene; 1,2,4-triethylbenzene; 1,2,3-trimethylbenzene, m-butyltoluene; p-butyltoluene; 3,5-diethyltoluene; o-ethyltoluene; p-ethyltoluene; m-propyltoluene; 4-ethyl-m-xylene; dimethylnaphthalenes; ethylnaphthalene; 2,3-dimethylanthracene; 9-ethylanthracene; 2-methylanthracene; o-methylanthracene; 9,10-dimethylphenanthrene; and 3-methyl-phenanthrene. Higher molecular weight alkylaromatic hydrocarbons can also be used as starting materials and include aromatic hydrocarbons such as are produced by the alkylation of aromatic hydrocarbons with olefin oligomers. Such products are frequently referred to in the art as alkylate and include hexylbenzene, nonylbenzene, dodecylbenzene, pentadecyclbenzene, hexyltoluene, nonyltoluene, dodecyltoluene and pentadecytoluene. Very often alkylate is obtained as a high boiling fraction in which the alkyl group attached to the aromatic nucleus varies in size from C₆ to C₁₂.

Reformate containing substantial quantities of benzene, toluene and/or xylene constitutes a particularly useful feed for the alkylation process of this invention.

Suitable alkylatable phenolic compounds include methylphenols (cresols); dimethylphenols (xylenols); ethyl, propyl and butylphenols; halophenols (e.g., chloro and bromo); alkylhalophenols; alkoxyphenols; dihydroxybenzens (e.g., hydroquinone catechol, resorcinol); and hydroxylated fused ring systems, e.g., naphtols, anthranols and phenanthranols.

In one embodiment of the invention, the alkylating agent is an organic compound having at least one available alkylating group capable of reaction with the alkylatable aromatic compound and possessing from 1 to 5 carbon atoms. Examples of suitable alkylating agents are C₂-C₅ olefins such as ethylene, propylene, the butenes and the pentenes; alcohols (inclusive of monoalcohols, dialcohols, trialcohols, etc.) such as methanol, ethanol, the propanols, the butanols and the pentanols; aldehydes such as formaldehyde, acetaldehyde, propionaldehyde, butyraldehyde and n-valeraldehyde; and alkyl halides such as methyl chloride, ethyl chloride, the propyl chlorides, the butyl chlorides and the pentyl chlorides.

Mixtures of light olefins are especially useful as alkylating agents in said one embodiment of this invention. Accordingly, mixtures of ethylene, propylene, butenes and/or pentenes which are major constituents of a variety of refinery streams, e.g., fuel gas, gas plant off-gas containing ethylene, propylene, etc., naphtha cracker off-gas containing light olefins, and refinery FCC propane/propylene streams, are useful alkylating agents herein. For example, a typical FCC light olefin stream possesses the following composition:

| | Wt.% | Mole% |
|---|---|---|
| Ethane | 3.3 | 5.1 |
| Ethylene | 0.7 | 1.2 |
| Propane | 14.5 | 15.3 |
| Propylene | 42.5 | 46.8 |
| Isobutane | 12.9 | 10.3 |
| n-Butane | 3.3 | 2.6 |
| Butenes | 22.1 | 18.32 |
| Pentanes | 0.7 | 0.4 |

In said one embodiment of the invention, useful products which can be obtained according to the process of the invention include ethylbenzene and cumene (by alkylation of benzene with ethylene and propylene, respectively), and alkylate reformate (by alkylation of reformate with fuel gas or other source of light olefins). In the case of benzene alkylation to produce ethylbenzene or cumene, it is found that the process of the invention results in less than 500 ppm of xylene by-product.

In another embodiment of the invention, the alkylating agent is an aliphatic or aromatic organic compound with one or more available alkylating aliphatic groups having at least 6 carbon atoms, preferably at least 8, and still more preferably at least 12 carbon atoms. Examples of suitable alkylating agents are olefins such as hexenes, heptenes, octenes, nonenes, decenes, undecenes and dodecenes; alcohols such as hexanols, heptanols, octanols, nonanols, decanols, undecanols and dodecanols; and alkyl halides such as hexyl chlorides, octyl chlorides and dodecyl chlorides. Branched alkylating agents, especially oligomerized olefins such as the trimers, tetramers and pentamers of light olefins such as ethylene, propylene and butylenes are also useful herein. Typical products of this further embodiment, particularly where the feedstock comprises benzene, toluene, xylene and or naphthalene, are aromatic lube base stocks of low pour and cloud point, high viscosity and good thermal and oxidative stability. Where the feedstock is a phenol, long chain alkylphenols, useful in the manufacture of synthetic detergents, can be obtained.

In its calcined form, the porous crystalline zeolite employed as the catalyst in the alkylation process of the invention has an X-ray diffraction pattern including the lines listed in Table I below:

**TABLE I**

| Interplanar d-Spacing (A) | Relative Intensity, I/Io x 100 |
|---|---|
| 12.36 ± 0.4 | M-VS |
| 11.03 ± 0.2 | M-S |
| 8.83 ± 0.14 | M-VS |
| 6.18 ± 0.12 | M-VS |
| 6.00 ± 0.10 | W-M |
| 4.06 ± 0.07 | W-S |
| 3.91 ± 0.07 | M-VS |
| 3.42 ± 0.06 | VS |

more specifically the lines listed in Table II below:

**TABLE II**

| Interplanar d-Spacing (A) | Relative Intensity, I/Io x 100 |
|---|---|
| 30.0 ± 2.2 | W-M |
| 22.1 ± 1.3 | W |
| 12.36 ± 0.4 | M-VS |
| 11.03 ± 0.2 | M-S |
| 8.83 ± 0.14 | M-VS |
| 6.18 ± 0.12 | M-VS |
| 6.00 ± 0.10 | W-M |
| 4.06 ± 0.07 | W-S |
| 3.91 ± 0.07 | M-VS |
| 3.42 ± 0.06 | VS |

and yet more specifically the lines listed in Table III below:

**TABLE III**

| Interplanar d-Spacing (A) | Relative Intensity, I/Io x 100 |
|---|---|
| 12.36 ± 0.4 | M-VS |
| 11.03 ± 0.2 | M-S |
| 8.83 ± 0.14 | M-VS |
| 6.86 ± 0.14 | W-M |
| 6.18 ± 0.12 | M-VS |
| 6.00 ± 0.10 | W-M |
| 5.54 ± 0.10 | W-M |
| 4.92 ± 0.09 | W |
| 4.64 ± 0.08 | W |
| 4.41 ± 0.08 | W-M |
| 4.25 ± 0.08 | W |
| 4.10 ± 0.07 | W-S |
| 4.06 ± 0.07 | W-S |
| 3.91 ± 0.07 | M-VS |
| 3.75 ± 0.06 | W-M |
| 3.56 ± 0.06 | W-M |
| 3.42 ± 0.06 | VS |
| 3.30 ± 0.05 | W-M |
| 3.20 ± 0.05 | W-M |
| 3.14 ± 0.05 | W-M |
| 3.07 ± 0.05 | W |
| 2.99 ± 0.05 | W |
| 2.82 ± 0.05 | W |
| 2.78 ± 0.05 | W |
| 2.68 ± 0.05 | W |
| 2.59 ± 0.05 | W |

Most specifically, the calcined zeolite has an X-ray diffraction pattern which includes the lines listed in Table IV below:

**TABLE IV**

| Interplanar d-Spacing (A) | Relative Intensity, I/Io x 100 |
|---|---|
| 30.0 ± 2.2 | W-M |
| 22.1 ± 1.3 | W |
| 12.36 ± 0.4 | M-VS |
| 11.03 ± 0.2 | M-S |
| 8.83 ± 0.14 | M-VS |
| 6.86 ± 0.14 | W-M |
| 6.18 ± 0.12 | M-VS |
| 6.00 ± 0.10 | W-M |
| 5.54 ± 0.10 | W-M |
| 4.92 ± 0.09 | W |
| 4.64 ± 0.08 | W |
| 4.41 ± 0.08 | W-M |
| 4.25 ± 0.08 | W |
| 4.10 ± 0.07 | W-S |
| 4.06 ± 0.07 | W-S |
| 3.91 ± 0.07 | M-VS |
| 3.75 ± 0.06 | W-M |
| 3.56 ± 0.06 | W-M |
| 3.42 ± 0.06 | VS |
| 3.30 ± 0.05 | W-M |
| 3.20 ± 0.05 | W-M |
| 3.14 ± 0.05 | W-M |
| 3.07 ± 0.05 | W |
| 2.99 ± 0.05 | W |
| 2.82 ± 0.05 | W |
| 2.78 ± 0.05 | W |
| 2.68 ± 0.05 | W |
| 2.59 ± 0.05 | W |

These values were determined by standard techniques. The radiation was the K-alpha doublet of copper and a diffractometer equipped with a scintillation counter and an associated computer was used. The peak heights, I, and the positions as a function of 2 theta, where theta is the Bragg angle, were determined using algorithms on the computer associated with the diffractometer. From these, the relative intensities, 100 I/Iₒ, where Iₒ is the intensity of the strongest line or peak, and d (obs.) the interplanar spacing in Angstroms Units (A), corresponding to the recorded lines, were determined. In Tables I-IV, the relative intensities are given in terms of the symbols W=weak, M=medium, S=strong and VS=very strong. In terms of intensities, these may be generally designated as follows:
- W: = 0 - 20
- M: = 20 - 40
- S: = 40 - 60
- VS: = 60 - 100

It should be understood that these X-ray diffraction patterns are characteristic of all species of the zeolite. The sodium form as well as other cationic forms reveal substantially the same pattern with some minor shifts in interplanar spacing and variation in relative intensity. Other minor variations can occur depending on the Y to X, e.g., silicon to aluminum, mole ratio of the particular sample, as well as its degree of thermal treatment.

The synthetic porous crystalline zeolite employed as the catalyst in the alkylation process of this invention, generally has a composition involving the molar relationship:

X₂O₃:(n)YO₂,

wherein X is a trivalent element, such as aluminum, boron, iron and/or gallium, preferably aluminum, Y is a tetravalent element such as silicon and/or germanium, preferably silicon, and n is at least 10, usually from 10 to 150, more usually from 10 to 60, and even more usually from 20 to 40. In the as-synthesized form, the zeolite has a formula, on an anhydrous basis and in terms of moles of oxides per n moles of YO₂, as follows:

(0.005-0.1)Na₂O:(1-4)R:X₂O₃:nYO₂

wherein R is an organic component. The Na and R components are associated with the zeolite as a result of their presence during crystallization, and are easily removed by post-crystallization methods hereinafter more particularly described.

The zeolite employed herein is thermally stable and exhibits high surface area (greater than 400 m²/gm as measured by the BET (Bruenauer, Emmet and Teller] test) and unusually large sorption capacity when compared to similar crystal structures. In particular, the zeolite exhibits Equilibrium sorption capacities greater than 4.5 wt.%, usually greater than 7 wt.%, for cyclohexane vapor and greater than 10 wt.% for n-hexane vapor and normally greater than 10 wt.% for water vapor. As is evident from the above formula, the present zeolite is synthesized nearly free of Na cations. It can, therefore, be used as an alkylation catalyst with acid activity without an exchange step. To the extent desired, however, the original sodium cations of the as-synthesized material can be replaced in accordance with techniques well known in the art, at least in part, by ion exchange with other cations. Preferred replacing cations include metal ions, hydrogen ions, hydrogen precursor, e.g., ammonium, ions and mixtures thereof. Particularly preferred cations are those which tailor the activity of the catalyst for alkylation. These include hydrogen, rare earth metals and metals of Groups IIA, IIIA, IVA, IB, IIB, IIIB, IVB and VIII of the Periodic Table of the Elements.

Prior to its use as alkylation catalyst, the zeolite should be subjected to thermal treatment to remove part or all of any organic constituent present therein.

The zeolite alkylation catalyst used herein can also be used in intimate combination with a hydrogenating component such as tungsten, vanadium, molybdenum, rhenium, nickel, cobalt, chromium, manganese, or a noble metal such as platinum or palladium where a hydrogenation-dehydrogenation function is to be performed. Such component can be introduced in the catalyst composition by way of cocrystallization, exchanged into the composition to the extent a Group IIIA element, e.g., aluminum, is in the structure, impregnated therein or intimately physically admixed therewith. Such component can be impregnated in, or on, the zeolite such as, for example, by, in the case of platinum, treating the zeolite with a solution containing a platinum metal-containing ion. Thus, suitable platinum compounds for this purpose include chloroplatinic acid, platinous chloride and various compounds containing the platinum amine complex.

Prior to its use in the alkylation process of this invention, the present zeolite crystals should be dehydrated, at least partially. This can be done by heating the zeolite to a temperature in the range of from 200°C to 595°C in an atmosphere such as air, nitrogen, etc. and at atmospheric, subatmospheric or superatmospheric pressures for between 30 minutes to 48 hours. Dehydration can also be performed at room temperature merely by placing the crystalline material in a vacuum, but a longer time is required to obtain a sufficient amount of dehydration.

The zeolite employed in the present process can be prepared from a reaction mixture containing sources of alkali or alkaline earth metal (M), e.g., sodium or potassium, cation, an oxide of trivalent element X, e.g, aluminum, an oxide of tetravalent element Y, e.g., silicon, an organic (R) directing agent, hexamethyleneimine, and water, said reaction mixture having a composition, in terms of mole ratios of oxides, within the following ranges:

| Reactants | Useful | Preferred |
|---|---|---|
| YO₂/X₂O₃ | 10 - 60 | 10 - 40 |
| H₂O/YO₂ | 5 - 100 | 10 - 50 |
| OH⁻/YO₂ | 0.01 - 1.0 | 0.1 - 0.5 |
| M/YO₂ | 0.01 - 2.0 | 0.1 - 1.0 |
| R/YO₂ | 0.05 - 1.0 | 0.1 - 0.5 |

In a preferred synthesis method, the YO₂ reactant contains a substantial amount of solid YO₂, e.g., at least about 30 wt.% solid YO₂. Where YO₂ is silica, the use of a silica source containing at least about 30 wt.% solid silica, e.g., Ultrasil (a precipitated, spray dried silica containing 90 wt.% silica) or HiSil (a precipitated hydrated SiO₂ containing 87 wt.% silica, 6 wt.% free H₂O and 4.5 wt.% bound H₂O of hydration and having a particle size of 0.02 micron) favors crystal formation from the above mixture. If another source of oxide of silicon, e.g., Q-Brand (a sodium silicate comprised of 28.8 wt.% of SiO₂, 8.9 wt.% Na₂O and 62.3 wt.% H₂O) is used, crystallization may yield little if any of the required zeolite and impurity phases of other crystal structures, e.g., ZSM-12, may be produced. Preferably, therefore, the YO₂, e.g., silica, source contains at least 30 wt.% solid YO₂, e.g., silica, and more preferably at least 40 wt.% solid YO₂, e.g., silica.

Crystallization of the required zeolite can be carried out at either static or stirred conditions in a suitable reactor vessel such as, e.g., polypropylene jars or teflon-lined or stainless steel autoclaves. Crystallization is generally conducted at a temperature of 80°C to 225°C for 25 hours to 60 days. Thereafter, the crystals are separated from the liquid and recovered.

Crystallization is facilitated by the presence of at least 0.01 percent, preferably 0.10 percent and still more preferably 1 percent, seed crystals based on total weight of the crystalline product.

Prior to use in the process of the invention the resultant zeolite is preferably combined with another material which is resistant to the temperatures and other conditions employed in the alkylation process of this invention. Such materials include active and inactive materials and synthetic or naturally occurring zeolites as well as inorganic materials such as clays, silica and/or metal oxides such as alumina. The latter may be either naturally occurring or in the form of gelatinous precipitates or gels including mixtures of silica and metal oxides. Use of a material in conjunction with the instant zeolite, i.e., combined therewith or present during its synthesis, which itself is catalytically active may change the conversion and/or selectivity of the catalyst. Inactive materials suitably serve as diluents to control the amount of conversion so that alkylation products can be obtained economically and orderly without employing other means for controlling the rate of reaction. These materials may be incorporated into naturally occurring clays, e.g., bentonite and kaolin, to improve the crush strength of the catalyst under commercial alkylation operating conditions. Said materials, i.e., clays, oxides, etc., function as binders for the catalyst. It is desirable to provide a catalyst having good crush strength because in commercial use, it is desirable to prevent the catalyst from breaking down into powder-like materials. These clay binders have been employed normally only for the purpose of improving the crush strength of the catalyst.

Naturally occurring clays which can be composited with the instant include the montmorillonite and kaolin family, which families include the subbentonites, and the kaolins commonly known as Dixie, McNamee, Georgia and Florida clays or others in which the main mineral constituent is halloysite, kaolinite, dickite, nacrite, or anauxite. Such clays can be used in the raw state as originally mined or initially subjected to calcination, acid treatment or chemical modification. Binders useful for compositing with the zeolite also include inorganic oxides, notably alumina.

In addition to the foregoing materials, the present zeolite can be composited with a porous matrix material such as silica-alumina, silica-magnesia, silica-zirconia, silica-thoria, silica-beryllia, silica-titania as well as ternary compositions such as silica-alumina-thoria, silica-alumina-zirconia silica-alumina-magnesia and silica-magnesia-zirconia. It may also be advantageous to provide at leat a part of the foregoing matrix materials in colloidal form so as to facilitate extrusion of the bound catalyst component(s).

The relative proportions of zeolite and inorganic oxide matrix vary widely, with the zeolite content ranging from 1 to 90 percent by weight and more usually, particularly when the composite is prepared in the form of beads, in the range of 2 to 80 weight percent of the composite.

The stability of the alkylation catalyst of the invention may be increased by steaming, which is conveniently effected by contacting the zeolite with 5-100% steam at a temperature of at least 300°C (preferably 300-650°C) for at least one hour (preferably 1-200 hours) at a pressure of 101-2,500 kPa. In a more particular embodiment, the catalyst can be made to undergo steaming with 75-100% steam at 315°-540°C and atmospheric pressure for 1-25 hours.

The alkylation process of the invention is generally conducted at a temperature of 0°C and 500°C., preferably 50°C. to 400°C, and most preferably 100 to 350°C; a pressure of 20 to 25350 kPa (0.2 to 250 atmospheres), preferably 100 to 2550 kPa (1 to 25 atmospheres); a molar ratio of alkylatable aromatic compound to alkylating agent of 0.1:1 to 50:1, preferably 0.5:1 to 10:1; and a feed weight hourly space velocity (WHSV) of 0.1 to 500, and preferably from 0.5 to 100, the latter WHSV being based upon the total weight of active catalyst (and binder if present).

The alkylation process of this invention can be carried out as a batch-type, semi-continuous or continuous operation utilizing a fixed, fluidized or moving bed catalyst system.

The invention will now be more particularly described with reference to the Examples and to the accompanying drawings, in which:
Figures 1-5 are X-ray diffraction patterns of the calcined crystalline material products of Examples 1, 3, 4, 5 and 7, hereinafter presented; and,
Figures 6-10 are graphical representations of process performance data relating to Example 15 described hereinafter.

In the Examples, whenever sorption data are set forth for comparison of sorptive capacities for water, cyclohexane and/or n-hexane, they were Equilibrium Adsorption values determined as follows:
A weighed sample of the calcined adsorbent was contacted with the desired pure adsorbate vapor in an adsorption chamber, evacuated to less than 1 mm Hg and contacted with 1.6 kPa (12 Torr) of water vapor or 5.3 kPa (40 Torr) of n-hexane or 5.3 kPa (40 Torr) cyclohexane vapor, pressures less than the vapor-liquid equilibrium pressure of the respective adsorbate at 90°C. The pressure was kept constant (within about ± 0.5 mm Hg) by addition of adsorbate vapor controlled by a manostat during the adsorption period, which did not exceed 8 hours. As adsorbate was adsorbed by the zeolite, the decrease in pressure caused the manostat to open a valve which admitted more adsorbate vapor to the chamber to restore the above control pressures. Sorption was complete when the pressure change was not sufficient to activate the manostat. The increase in weight was calculated as the adsorption capacity of the sample in g/100 g of calcined adsorbant. The zeolite employed in the process of the invention always exhibits Equilibrium Adsorption values of greater than 4.5 wt.%, usually greater than 7 wt.% for cyclohexane vapor and greater than 10 wt.% for n-hexane vapor and normally greater than 10 wt.% for water vapor.

When Alpha Value is examined, it is noted that the Alpha Value is an approximate indication of the catalytic cracking activity of the catalyst compared to a standard catalyst and it gives the relative rate constant (rate of normal hexane conversion per volume of catalyst per unit time). It is based on the activity of a highly active silica-alumina cracking catalyst taken as an Alpha of 1 (Rate Constant = 0.016 sec⁻¹). The Alpha Test which was used herein is described in J. Catalysis, 61, pp. 390-396 (1980).

### EXAMPLE 1

1 part of sodium aluminate (43.5% Al₂O₃, 32.2% Na₂O, 25.6% H₂O) was dissolved in a solution containing 1 part of 50% NaOH solution and 103.13 parts H₂O. To this was added 4.50 parts hexamethyleneimine. The resulting solution was added to 8.55 parts of Ultrasil, a precipitated, spray-dried silica (about 90% SiO₂).

The reaction mixture had the following composition, in mole ratios:

| | |
|---|---|
| SiO₂/Al₂O₃ | = 30.0 |
| OH⁻/SiO₂ | = 0.18 |
| H₂O/SiO₂ | = 44.9 |
| Na/SiO₂ | = 0.18 |
| R/SiO₂ | = 0.35 |

where R is hexamethyleneimine.

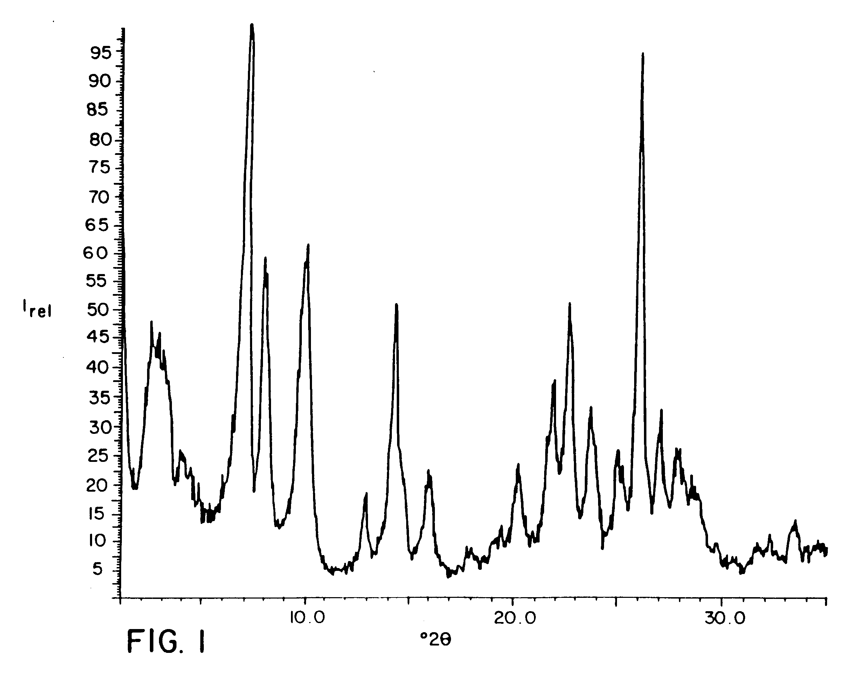

The mixture was crystallized in a stainless steel reactor, with stirring, at 150°C for 7 days. The crystalline product was filtered, washed with water and dried at 120°C. After a 20 hour calcination at 538°C, the X-ray diffraction pattern contained the major lines listed in Table V. Figure 1 shows the X-ray diffraction pattern of the calcined product. The sorption capacities of the calcined material were measured to be:

| | |
|---|---|
| H₂O | 15.2 wt.% |
| Cyclohexane | 14.6 wt.% |
| n-Hexane | 16.7 wt.% |

The surface area of the calcined crystalline material was measured to be 494 m²/g.

The chemical composition of the uncalcined material was determined to be as follows:

| Component | wt.% |
|---|---|
| SiO₂ | 66.9 |
| Al₂O₃ | 5.40 |
| Na | 0.03 |
| N | 2.27 |
| Ash | 76.3 |
| SiO₂/Al₂O₃, mole ratio - 21.1 | |

**TABLE V**

| Degrees 2-Theta | Interplanar d-Spacing (A) | I/Iₒ |
|---|---|---|
| 2.80 | 31.55 | 25 |
| 4.02 | 21.98 | 10 |
| 7.10 | 12.45 | 96 |
| 7.95 | 11.12 | 47 |
| 10.00 | 8.85 | 51 |
| 12.90 | 6.86 | 11 |
| 14.34 | 6.18 | 42 |
| 14.72 | 6.02 | 15 |
| 15.90 | 5.57 | 20 |
| 17.81 | 4.98 | 5 |
| 20.20 | 4.40 | 20 |
| 20.91 | 4.25 | 5 |
| 21.59 | 4.12 | 20 |
| 21.92 | 4.06 | 13 |
| 22.67 | 3.92 | 30 |
| 23.70 | 3.75 | 13 |
| 24.97 | 3.57 | 15 |
| 25.01 | 3.56 | 20 |
| 26.00 | 3.43 | 100 |
| 26.69 | 3.31 | 14 |
| 27.75 | 3.21 | 15 |
| 28.52 | 3.13 | 10 |
| 29.01 | 3.08 | 5 |
| 29.71 | 3.01 | 5 |
| 31.61 | 2.830 | 5 |
| 32.21 | 2.779 | 5 |
| 33.35 | 2.687 | 5 |
| 34.61 | 2.592 | 5 |

### EXAMPLE 2

A portion of the calcined crystalline product of Example 1 was tested in the Alpha Test and was found to have an Alpha Value of 224.

### EXAMPLES 3-5

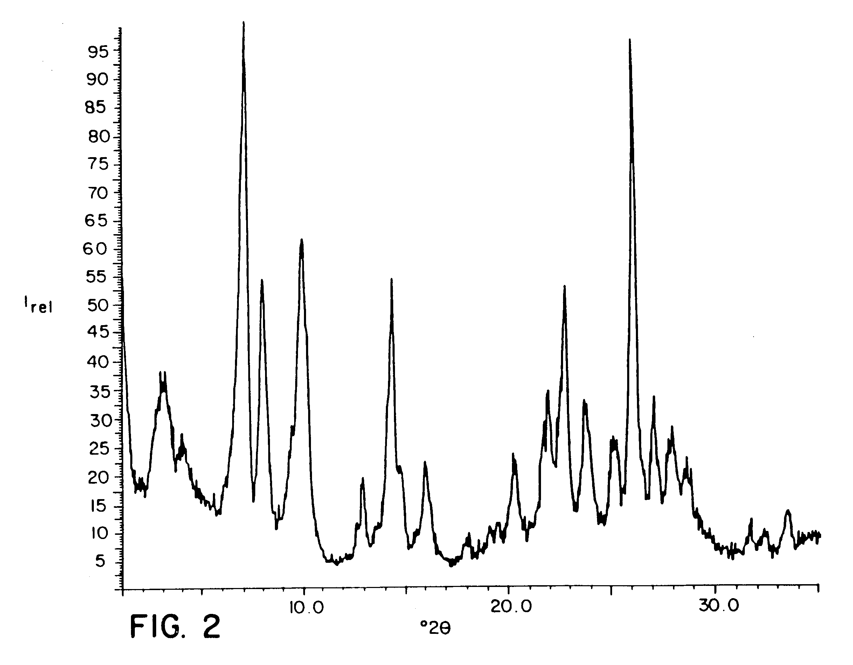

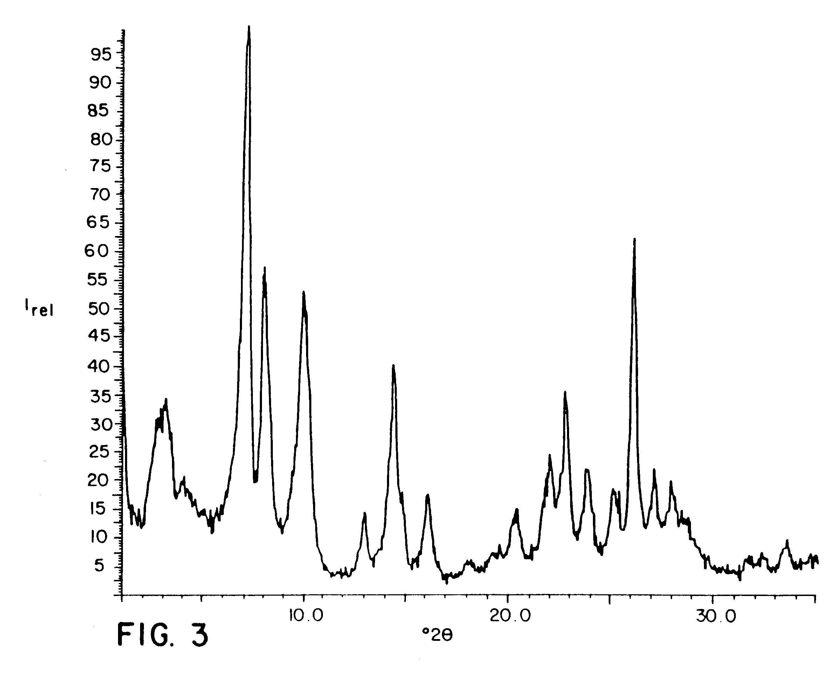

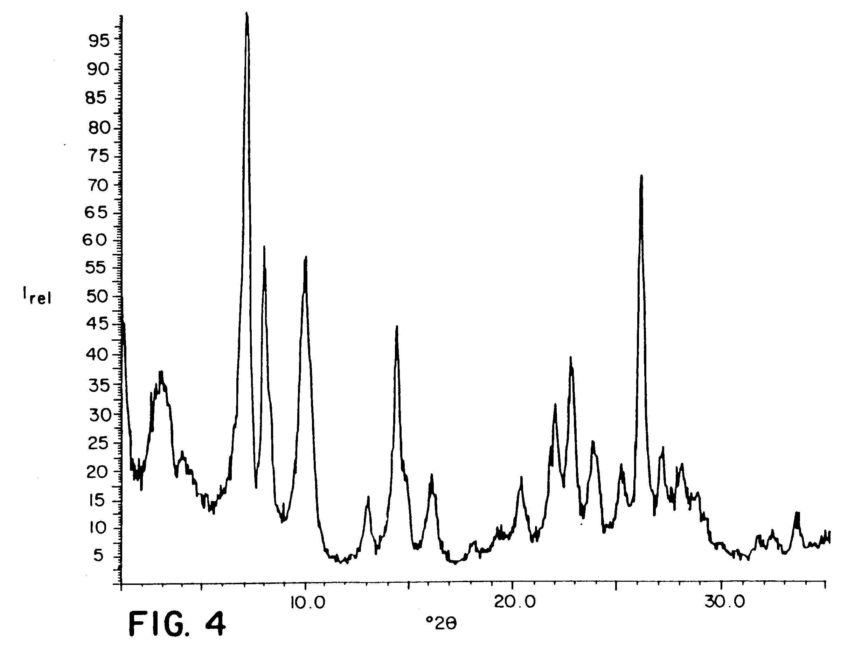

Three separate synthesis reaction mixtures were prepared with compositions indicated in Table VI. The mixtures were prepared with sodium aluminate, sodium hydroxide, Ultrasil, hexamethyleneimine (R) and water. The mixtures were maintained at 150°C, 143°C and 150°C, respectively, for 7, 8 and 6 days respectively in stainless steel autoclaves at autogenous pressure. Solids were separated from any unreacted components by filtration and then water washed, followed by drying at 120°C. The product crystals were subjected to X-ray diffraction, sorption, surface area and chemical analyses. The results of the sorption, surface area and chemical analyses are presented in Table VI and the X-ray diffraction patterns are presented in Figures 2, 3 and 4, respectively. The sorption and surface area measurements were of the calcined product.

**TABLE VI**

| Example | 3 | 4 | 5 |
|---|---|---|---|
| Synthesis Mixture, mole ratios | | | |
| SiO₂/Al₂O₃ | 30.0 | 30.0 | 30.0 |
| OH⁻/SiO₂ | 0.18 | 0.18 | 0.18 |
| H₂O/SiO₂ | 19.4 | 19.4 | 44.9 |
| Na/SiO₂ | 0.18 | 0.18 | 0.18 |
| R/SiO₂ | 0.35 | 0.35 | 0.35 |

| Product Composition, Wt.% | | | |
|---|---|---|---|
| SiO₂ | 64.3 | 68.5 | 74.5 |
| Al₂O₃ | 4.85 | 5.58 | 4.87 |
| Na | 0.08 | 0.05 | 0.01 |
| N | 2.40 | 2.33 | 2.12 |
| Ash | 77.1 | 77.3 | 78.2 |
| SiO₂/Al₂O₃, mole ratio | 22.5 | 20.9 | 26.0 |

| Adsorption, Wt.% | | | |
|---|---|---|---|
| H₂O | 14.9 | 13.6 | 14.6 |
| Cyclohexane | 12.5 | 12.2 | 13.6 |
| n-Hexane | 14.6 | 16.2 | 19.0 |
| Surface Area, n²/g | 481 | 492 | 487 |

### EXAMPLE 6

Quantities of the calcined (538°C for 3 hours) crystalline silicate products of Examples 3, 4 and 5 were tested in the Alpha Test and found to have Alpha Values of 127, 180 and 187, respectively.

### EXAMPLE 7

To demonstrate a further preparation of the present zeolite, 4.49 parts of hexamethyleneimine was added to a solution containing 1 part of sodium aluminate, 1 part of 50% NaOH solution and 44.19 parts of H₂O. To the combined solution were added 8.54 parts of Ultrasil silica. The mixture was crystallized with agitation at 145°C for 59 hours and the resultant product was water washed and dried at 120°C.

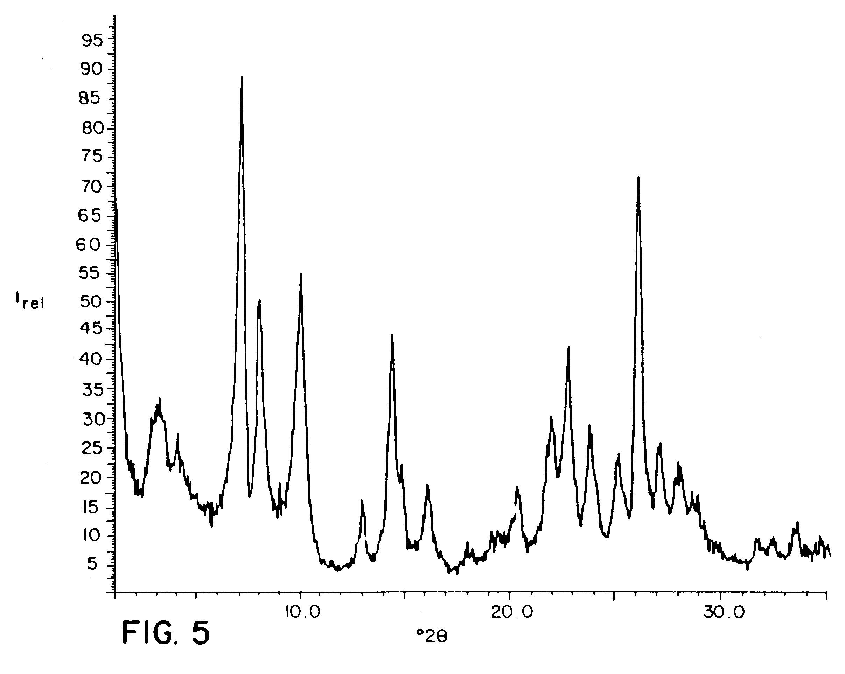

The X-ray diffraction pattern of the dried product crystals is presented in Figure 5 and demonstrates the product to be the crystalline material of this invention. Product chemical composition, surface area and adsorption analyses results were as set forth in Table VII:

**TABLE VII**

| Product Composition (uncalcined) | |
|---|---|
| C | 12.1 wt.% |
| N | 1.98 wt.% |
| Na | 640 ppm |
| Al₂O₃ | 5.0 wt.% |
| SiO₂ | 74.9 wt.% |
| SiO₂/Al₂O₃, mole ratio | 25.4 |

| Adsorption, wt.% | |
|---|---|
| Cyclohexane | 9.1 |
| N-Hexane | 14.9 |
| H₂O | 16.8 |
| Surface Area, m²/g | 479 |

### EXAMPLE 8

25g grams of solid crystal product from Example 7 were calcined in a flowing nitrogen atmospheres at 538°C for 5 hours, followed by purging with 5% oxygen gas (balance N₂) for another 16 hours at 538°C.

Individual 3g samples of the calcined material were ion-exchanged with 100 ml of 0.1N TEABr, TPABr and LaCl₃ solution separately. Each exchange was carried out at ambient temperature for 24 hours and repeated three times. The exchanged samples were collected by filtration, water-washed to be halide-free and dried. The compositions of the exchanged samples are tabulated below demonstrating the exchange capacity of the present crystalline silicate for different ions.

| Exchange Ions Ionic Composition, wt.% | TEA | TPA | La |
|---|---|---|---|
| Na | 0.095 | 0.089 | 0.063 |
| N | 0.30 | 0.38 | 0.03 |
| C | 2.89 | 3.63 | - |
| La | - | - | 1.04 |

### EXAMPLE 9

The La-exchanged sample from Example 8 was sized to 14 to 25 mesh and then calcined in air at 538°C for 3 hours. The calcined material had an Alpha Value of 173.

### EXAMPLE 10

The calcined sample La-exchanged material from Example 9 was severely steamed at 649°C in 100% steam for 2 hours. The steamed sample had an Alpha Value of 22, demonstrating that the zeolite has very good stability under severe hydrothermal treatment.

### EXAMPLE 11

This example illustrates the preparation of the present zeolite where X in the general formula, supra, is boron. Boric acid, 2.59 parts, was added to a solution containing 1 part of 45% KOH solution and 42.96 parts H₂O. To this was added 8.56 parts of Ultrasil silica, and the mixture was thoroughly homogenized. A 3.88 parts quantity of hexamethyleneimine was added to the mixture.

The reaction mixture had the following composition in mole ratios:

| | |
|---|---|
| SiO₂/B₂O₃ | = 6.1 |
| OH⁻/SiO₂ | = 0.06 |
| H₂O/SiO₂ | = 19.0 |
| K/SiO₂ | = 0.06 |
| R/SiO₂ | = 0.30 |

where R is hexamethyleneimine.

The mixture was crystallized in a stainless steel reactor, with agitation, at 150°C for 8 days. The crystalline product was filtered, washed with water and dried at 120°C. A portion of the product was calcined for 6 hours at 540°C and found to have the following sorption capacities:

| | |
|---|---|
| H₂O (12 Torr) | 11.7 wt.% |
| Cyclohexane (40 Torr) | 7.5 wt.% |
| n-Hexane (40 Torr) | 11.4 wt.% |

The surface area of the calcined crystalline material was measured (BET) to be 405m²/g.

The chemical composition of the uncalcined material was determined to be as follows:

| | |
|---|---|
| N | 1.94 wt.% |
| Na | 175 ppm |
| K | 0.60 wt.% |
| Boron | 1.04 wt.% |
| Al₂O₃ | 920 ppm |
| SiO₂ | 75.9 wt.% |
| Ash | 74.11 wt.% |
| SiO₂/Al₂O₃, molar ratio | = 1406 |
| SiO₂/(Al+B)₂O₃, molar ratio | = 25.8 |

### EXAMPLE 12

A portion of the calcined crystalline product of Example 11 was treated with NH₄Cl and again calcined. The final crystalline product was tested in the Alpha Test and found to have an Alpha Value of 1.

### EXAMPLE 13

This example illustrates another preparation of the zeolite in which X of the general formula, supra, is boron. Boric acid, 2.23 parts, was added to a solution of 1 part of 50% NaOH solution and 73.89 parts H₂O. To this solution was added 15.29 parts of HiSil silica followed by 6.69 parts of hexamethyleneimine. The reaction mixture had the following composition in mole ratios:

| | |
|---|---|
| SiO₂/B₂O₃ | = 12.3 |
| OH⁻/SiO₂ | = 0.056 |
| H₂O/SiO₂ | = 18.6 |
| K/SiO₂ | = 0.056 |
| R/SiO₂ | = 0.30 |

where R is hexamethyleneimine.

The mixture was crystallized in a stainless steel reactor, with agitation, at 300°C for 9 days. The crystalline product was filtered, washed with water and dried at 120°C. The sorption capacities of the calcined material (6 hours at 540°C) were measured:

| | |
|---|---|
| H₂O (12 Torr) | 14.4 wt.% |
| Cyclohexane (40 Torr) | 4.6 wt.% |
| n-Hexane (40 Torr) | 14.0 wt.% |

The surface area of the calcined crystalline material was measured to be 438m²/g.

The chemical composition of the uncalcined material was determined to be as follows:

| Component | Wt.% |
|---|---|
| N | 2.48 |
| Na | 0.06 |
| Boron | 0.83 |
| Al₂O₃ | 0.50 |
| SiO₂ | 73.4 |
| SiO₂/Al₂O₃, molar ratio | = 249 |
| SiO₂/(Al+B)₂O₃, molar ratio | = 28.2 |

### EXAMPLE 14

A portion of the calcined crystalline product of Example 13 was tested in the Alpha Test and found to have an Alpha Value of 5.

### EXAMPLE 15

Comparative catalyst aging runs for the alkylation of benzene with propylene were carried out with zeolite of the invention and with ZSM-12 at process conditions of 17 hr⁻¹ benzene WHSV, 3 to 1 mole ratio benzene to propylene and 2170 kPa (300 psig).

The zeolite of the invention was prepared by adding 4.49 parts quantity of hexamethyleneimine to a mixture containing 1.00 part sodium aluminate, 1.00 part 50% NaOH, 8.54 parts Ultrasil VN3 and 44.19 parts deionized H₂O. The reaction mixture was heated to 143°C (290°F) and stirred in an autoclave at that temperature for crystallization. After full crystallinity was achieved, the majority of the hexamethyleneimine was removed from the autoclave by controlled distillation and the zeolite crystals separated from the remaining liquid by filtration, washed with deionized H₂O and dried.

A portion of the zeolite crystals was combined with Al₂O₃ to form a mixture of 65 parts, by weight, zeolite and 35 parts Al₂O₃. Water was added to this mixture to allow the resulting catalyst to be formed into extrudates. The catalyst was activated by calcining in nitrogen at 540°C (1000°F), followed by aqueous ammonium nitrate exchange and calcining in air at 540°C (1000°F).

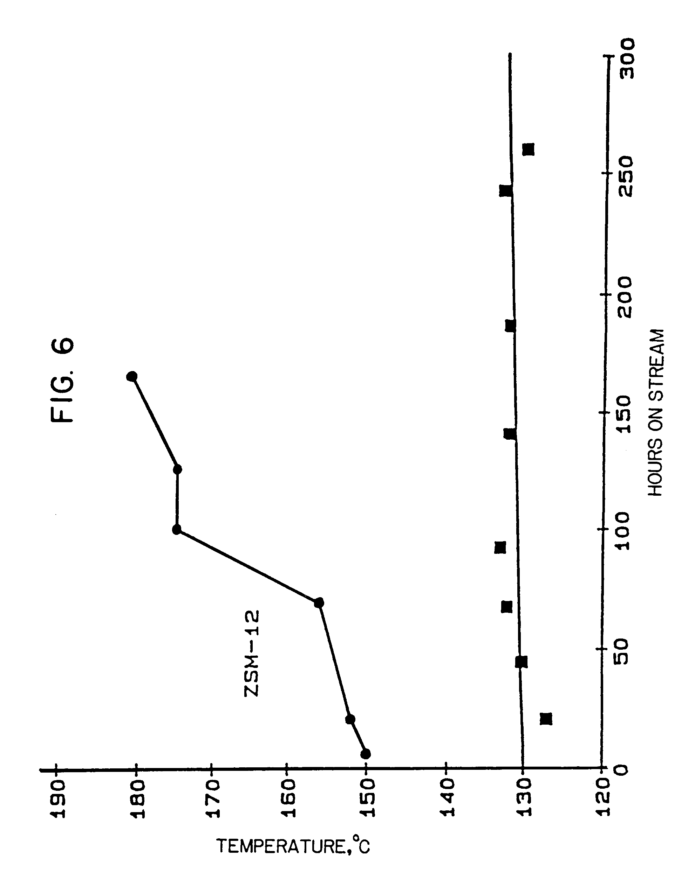

Figure 6 shows the temperature required to maintain complete propylene conversion. At 130°C, the present zeolite does not age during 270 stream hours under isothermal reaction conditions.

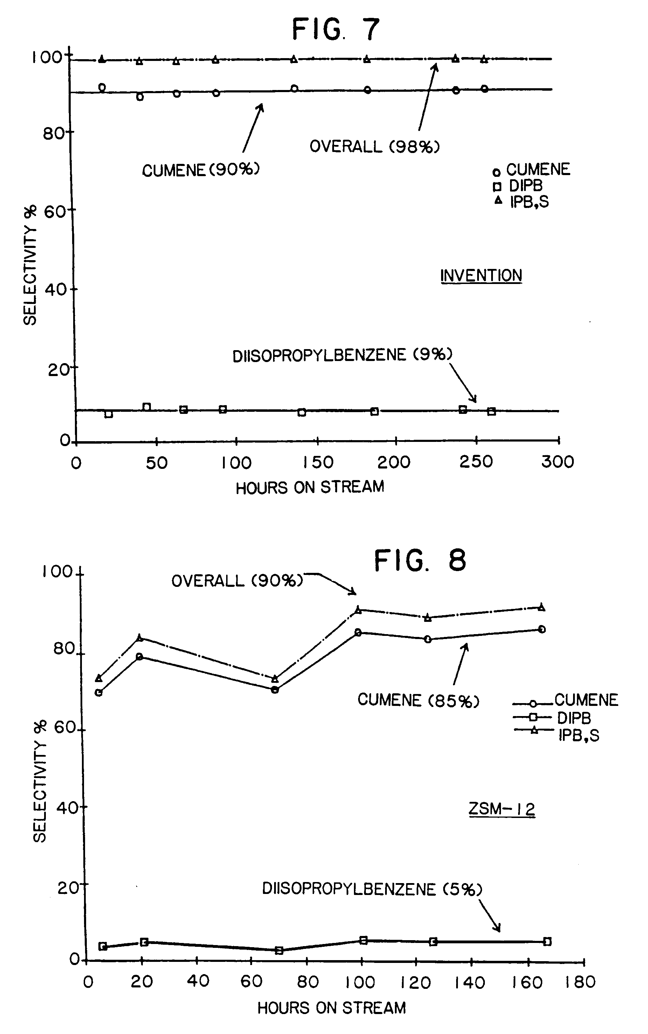

Selectivity to isopropylbenzenes (IPBs) for the zeolite of the invention and for ZSM 12 is shown in Figures 7 and 8 respectively. Using the present zeolite, overall selectivity to IPBs is approximately 100% compared to 90% using ZSM-12 under conditions of complete propylene conversion. This and chromatographic evidence indicate that propylene is oligomerizing over ZSM-12 leading to lower IPB selectivity.

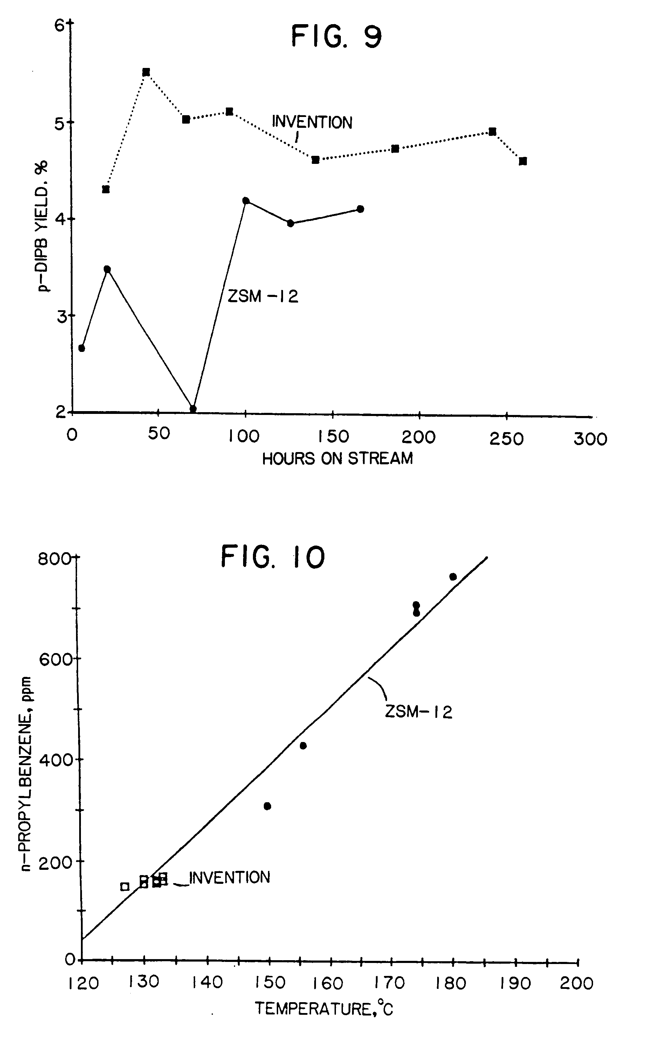

The data plotted in Figure 9 show that zeolite of the invention is more active for the formation of diisopropylbenzenes (DIPB) than ZSM-12. Thus, when alkylating benzene with propylene, 10% of the products over the present zeolite are DIPBs, primarily the meta and para isomers. Figure 9 shows that the yield of para-DIPB is greater over the present zeolite (about 5 wt.% of total hydrocarbon product) than over ZSM-12 (about 4%). DIPBs are intermediates in the production of dihydroxybenzenes such as hydroquinone (p-) and resorcinol (m-), both of which have important industrial uses.

Figure 10 shows how the ratio of n-propylbenzene to cumene in the reaction products over ZSM-12 and the present zeolite varies with reaction temperature. Over 270 hours of reaction with the present zeolite, the n-propylbenzene to cumene ratio remained approximately constant at 160 ppm. This compares to a level of 700 ppm for ZSM-12 at the same condition of 98%+ propylene conversion but at higher temperature.

### EXAMPLE 16

This example illustrates the alkylation of cumene with propylene in the presence of the present zeolite to provide diisopropylbenzenes (DIPBs). Alkylation reaction conditions were 2170 kPa (300 psig), 150°C and a 1:1 mole ratio of cumene to propylene. An 81% conversion of cumene to alkylate was achieved. DIPBs comprised 84% of this alkylated product with the remainder being triisopropylbenzene (TIPB). The DIPBs are para (65%), meta (34%) and ortho (1%).

### EXAMPLE 17

This example demonstrates the alkylation of phenol with alpha-C₁₄ olefin over the present zeolite to provide a mixture of alkylated phenols. Alkylation was carried out in a 1 liter autoclave using 400 grams (2.02 moles) olefin, 95 grams (1.01 moles) phenol and 38 grams catalyst, 65 wt.% MCM-22/35 wt.% Al₂O₃ binder. Reaction time was six hours at 177°C (350°F) under 2860 kPa (400 psig) nitrogen.

Analysis of the product indicated the presence of mono-, di- and tri-tetradecyl phenols.

### EXAMPLE 18

This example shows the alkylation of benzene with 1-dodecene employing each of two known alkylation catalysts, namely, the Lewis acid AlCl₃ and zeolite Beta, such being disclosed in U.S. Patent No. 4,301,316. The isomer distributions are shown in Table VIII as follows:

**TABLE VIII**

| 1-Dodecene Alkylation Isomer Distribution, Wt.% | | |
|---|---|---|
| Alkybenzene Isomer | AlCl₃ | Zeolite Beta |
| 2 | 30 | 57 |
| 3 | 19 | 18 |
| 4 | 17 | 10 |
| 5 | 17 | 7 |
| 6 | 17 | 8 |

The composition of the dodecylbenzene mixture is to some extent dependent upon the acid catalyst involved. Sulfuric acid has been reported to result in 41 wt.% 2-dodecylbenzene while HF yields only 20 wt.% Similar results can be shown for other alkylations involving relatively large, i.e., C₆+, alkylating agents.

### EXAMPLE 19

This example shows the alkylation of benzene with alpha-C₁₄ olefin (Shell's Neodene-14) over the zeolite of the invention (produced according to Example 15) and separately over zeolite Beta. Alkylation was carried out in a 1 liter autoclave using 400 grams (2.02 moles) olefin, 79 grams (1.01 moles) benzene and 38 grams of catalyst. Reaction time was six hours at 204°C (400°F) under 2860 kPa (400 psig) nitrogen. The isomer distributions are shown in Table IX as follows:

**TABLE IX**

| Alkylation Isomer Distribution, Wt.% | | |
|---|---|---|
| Alkybenzene Isomer | Zeolite of the invention | Zeolite Beta |
| 2 | 59.2 | 54.7 |
| 3 | 36.5 | 20.3 |
| 4 | 2.5 | 9.4 |
| 5 | 0.9 | 5.8 |
| 6 | 0.4 | 5.3 |
| 7 | 0.5 | 5.5 |

As the data in Examples 18 and 19 show, the use of the zeolite in accordance with this invention as alkylation catalyst results in a significantly higher percentage of alkylated product of the 2- and 3-alkyl isomer variety than a known Lewis acid or zeolite Beta alkylation catalyst under identical or similar conditions.

The alkylated products possessing alkyl side chains of approximately 8 to 16 carbon atoms are especially useful as intermediates for the production of linear alkylbenzene sulfonate synthetic detergents.

### EXAMPLE 20

Two different zeolite catalysts, the zeolite of the invention produced according to Example 15, and zeolite beta were used in separate alkylation runs, A and B, carried out under essentially identical conditions to provide lube base stocks. Each catalyst composition contained with 65% zeolite bound with 35 wt.% alumina.

The alkylation reaction for each run was carried out in a 1 liter autoclave using 400g (2.02 moles) of alpha C₁₄ olefin (Shell Neodene-14), 79g (1.01 moles) of benzene (a 5:1 mole ratio of benzene to olefin) with 38g catalyst at 204°C (400°F) for 6 hours under the nitrogen pressure of 2860 kPa (400 psig).

Table X below sets forth the lube yield and lube properties resulting from alkylations carried out with each of the foregoing zeolites.

**TABLE X**

| Catalyst | Run A | Run B |
|---|---|---|
| | Example 15 Invention | Beta |
| Lube yield, wt.% | 77.0 | 37.0 |

| Lube Properties: | | |
|---|---|---|
| Pour Point, °F(°C) | -60(-51) | -60(-51) |
| Cloud Point, °F(°C) | -38(-39) | -50(-46) |
| KV at 40°C, cSt | 12.59 | 14.54 |
| KV at 100°C, cSt | 3.151 | 3.471 |
| VI | 113 | 117 |

Gas Chromatographic and Field Ionization Mass Spectrographic (FIMS) analysis indicated that the synthetic lube produced from the catalyst of the invention contained a mixture of mono- and di-alkyl benzene compounds, 67 and 33 wt.%, respectively. The other catalyst, i.e., zeolite Beta, promoted not only alkylation to form mono- and di-alkyl benzenes but also C₁₄ oligomerization to form C₂₈ olefins. In addition to exhibiting a unique alkylation selectivity, the catalyst of the invention is significantly more active and produces alkylated benzene lube base stock with very low pour and cloud point compared to those of zeolite Beta.

### EXAMPLE 21

This example describes the use of the catalyst of the invention in the alkylation of naphthalene with an alpha C₁₄ olefin. The reaction was carried out under process conditions similar to those in Example 20 using a 0.5:1 mole ratio of alpha olefin to naphthalene. The alkylated naphthalene lube yield was about 94 wt.% and the product contained synthetic lubes contain predominantly a mixture of mono-, di- and tri-alkyl naphthalenes and have the following properties (Table XI):

**TABLE XI**

| Catalyst | Zeolite of the invention |
|---|---|
| Lube yield, wt.% | 94 |

| Lube Properties | |
|---|---|
| Pour Point, °F(°C) | <-65 ( -54) |
| KV at 40°C, cSt | 37.27 |
| KV at 100°C, cSt | 5.894 |
| VI | 100 |

### EXAMPLE 22

This example also illustrates the excellent activity and selectivity of the present zeolite catalyst for alkylating alpha C₁₄ olefin with other aromatics such as toluene (Example 22A) and xylene (Example 22B) as compared to benzene (Example 22C) under similar process conditions (Table XII):

**TABLE XII**

| Example No. | 22A | 22B | 22C |
|---|---|---|---|
| Aromatics | Toluene | Xylene | Benzene |
| Olefins | C₁₄ | C₁₄ | C₁₄ |
| Mole Ratios | | | |
| C₁₄/Aromatics | 1 | 1 | 1 |
| Lube Yield, wt.% | 92.0 | 88.6 | 73.0 |

| Lube Properties | | | |
|---|---|---|---|
| Pour Point, °F(°C) | <-65(<-54) | <-65(<-54) | -45(-43) |
| Cloud Point, °F(°C) | -65(-54) | -52(-47) | -44(-42) |
| KV at 40°C, cSt | 9.408 | 16.13 | 7.651 |
| KV at 100°C, cSt | 2.505 | 3.393 | 2.265 |
| VI | 87 | 70 | 106 |

### EXAMPLE 23

This example illustrates an alkylation process in accordance with this invention which utilizes an olefinic feedstock obtained from the oligomerization of 1-decene employing propanol-promoted BF₃ catalyst. The catalyst employed was the zeolite of the invention produced as in Example 15, including combination with the Al₂O₃ binder and conversion to the hydrogen form.

1-Decene, BF₃ and propanol were introduced into a reactor to oligomerize the 1-decene. The oligomerized product was washed firstly with sodium hydroxide and then with water, prior to separation of light products therefrom in a vacuum distillation unit. 250g of the 1-decene oligomers, containing 33 wt.% C₃₀ olefin, 52 wt.% C₄₀ olefin and 15 wt.% C₅₀ olefin, was then used to alkylate 78g of benzene employing 22g of the zeolite of the invention as the catalyst. The reaction was carried out at 2860 kPa (400 psig) nitrogen and 204°C (400°F) for 6 hours.

After decanting the catalyst and distilling off any unreacted benzene, the lube yield was 88 wt.% indicating that 12 wt.% benzene was alkylated and incorporated into the backbone structure of the decene oligomers. This was further confirmed by IR analysis. The properties of the oligomers before and after alkylation of the benzene are shown as follows (Table XIII):

**TABLE XIII**

| PROCESS | OLIGOMERIZATION | ALKYLATION |
|---|---|---|
| Lube Properties | | |
| Pour Point, °F(°C) | <-65 (<-54) | <-65 (<-54) |
| Cloud Point, °F(°C) | <-65 (<-54) | < -65 (<-54) |
| KV at 40°C, cSt | 25.73 | 33.03 |
| KV at 100°C, cSt | 5.225 | 6.039 |
| VI | 138 | 131 |

| Product Quality | | |
|---|---|---|
| Thermal Stability at 288°C (550°F)% Vis. Decrease | 10.9 | 4.6 |
| B-10 Oxidative Stability % Viscosity Increase | 120 | 80.6 |
| DSC-IP minutes at 180°C | 5.0 | 10.5 |

The results indicate that the alkylation step produces a benzene-containing synthetic lube base stock with excellent product properties such as very low pour and cloud point and high Viscosity Index together with improved additive solvency characteristics as well as enhanced thermal and oxidative stability.

### EXAMPLE 24

This example illustrates the alkylation process of this invention carried out with a 1-decene oligomer product obtained with a Cr/SiO₂ catalyst.

Thus, 1-decene and Cr/SiO₂ were introduced into oligomerization reactor with the product therefrom being vacuum stripped and thereafter being introduced into an alkylation reactor together with benzene.

The alkylation reaction was carried out under identical process conditions as in Example 23 but using 500g decene oligomers and 95g of benzene with 36g of zeolite of the invention as catalyst. The properties of the decene oligomers before and after alkylation of the benzene are shown as follows (Table XIV):

**TABLE XIV**

| PROCESS | OLIGOMERIZATION | ALKYLATION |
|---|---|---|
| Lube Properties | | |
| Pour Point, °F(°C) | -30 (-34) | -25 (-32) |
| Cloud Point, °F(°C) | <-65 (<-54) | -30 (-34) |
| KV at 40°C, cSt | 122.9 | 68.11 |
| KV at 100°C, cSt | 18.33 | 11.60 |
| VI | 167 | 166 |

### EXAMPLE 25

In a manner similar to Example 23, 400g of 204-371°C (400-700°F) distillate (78 wt.%), prepared by oligomerizating light olefins over ZSM-5, was alkylated with 115g naphthalene (12 wt.%) over the catalyst of the invention. The resulting 370°C+ (700°F+) lube yield was 54 wt.%. Table XV shows the properties of the alkylated naphthalene lube base stock:

**TABLE XV**

| PROCESS | OLEFIN CONVERSION TO GASOLINE AND DISTILLATE | ALKYLATION |
|---|---|---|
| Properties | | |
| Pour Point, °F(°C) | <-65 (<-54) | 0(-18) |
| KV at 40°C, cSt | --- | 152.6 |
| KV at 100°C, cSt | 2.5 | 10.15 |

| Sim. Dist., °F(°C) | | |
|---|---|---|
| IBP/5% | 300/375(149/191) | 636/679(336/359) |
| 10/20% | 435/467(224/242) | 701/732(372/389) |
| 30/40% | 488/509(253/265) | 754/776(401/413) |
| 50% | 529(276) | 799(426) |
| 60/70% | 553/583(289/306) | 825/856(441/458) |
| 80/90% | 622/679(328/354) | 894/948(479/509) |
| 95% | 725(385) | 990(532) |

## Claims

1. A process for alkylating an aromatic compound comprising contacting the aromatic compound with at least one alkylating agent in the presence of a catalyst comprising a synthetic porous crystalline zeolite having an X-ray diffraction pattern including values substantially as set forth in Table I of the specification.

2. The process of Claim 1 wherein the zeolite has an X-ray diffraction pattern including values substantially as set forth in Table II of the specification.

3. The process of Claim 1 wherein the zeolite has an X-ray diffraction pattern including values substantially as set forth in Table III of the specification.

4. The process of Claim 1 wherein the zeolite has an X-ray diffraction pattern including values substantially as set forth in Table IV of the specification.

5. The process of Claim 1 wherein the zeolite has a composition comprising the molar relationship
X₂O₃:(n)YO₂,
wherein n is at least about 10, X is a trivalent element and Y is a tetravalent element.

6. The process of Claim 5 wherein X comprises aluminum and Y comprises silicon.

7. The process of Claim 1 wherein the zeolite has equilibrium adsorption capacities greater than 4.5 wt.% for cyclohexane vapor and greater than 10 wt.% for n-hexane vapor.

8. The process of Claim 1 wherein the alkylating agent comprises at least one unsaturated, aliphatic group having 2 to 5 carbon atoms.

9. The process of Claim 8 wherein the alkylating agent is propylene and the aromatic compound is benzene and/or cumene.

10. The process of Claim 8 wherein the alkylating agent is ethylene and the aromatic compound is benzene.

11. The process of Claim 1 wherein the alkylating agent comprises at least one aliphatic chain having at least 6 carbon atoms.

12. The process of Claim 11 wherein the aromatic compound is a hydrocarbon or a phenolic compound.

13. The process of Claim 1 wherein the alkylation conditions include a temperature of 0°C to 500°C, a pressure of 20 to 25350 kPa (0.2 to 250 atmospheres), a WHSV of 0.1 to 500 and a molar ratio of alkylatable aromatic compound to alkylating agent of 0.1:1 to 50:1.

14. The process of Claim 1 wherein the alkylation conditions include a temperature of 10°C to 350°C, a pressure of 100 to 2550 kPa (1 to 25 atmospheres), a WHSV of 0.5 to 100 and a molar ratio of alkylatable aromatic compound to alkylating agent of 0.5:1 to 10:1.

## Patentansprüche

1. Verfahren zur Alkylierung einer aromatischen Verbindung, welches umfaßt, daß die aromatische Verbindung mit wenigstens einem Alkylierungsmittel in Gegenwart eines Katalysators in Kontakt gebracht wird, der einen synthetischeen porösen kristallinen Zeolith umfaßt, der ein Röntgenbeugungsmuster einschließlich der in Tabelle I der Beschreibung angegebenen Linien aufweist.

2. Verfahren nach Anspruch 1, worin der Zeolith ein Röntgenbeugungsmuster einschließlich der Werte, wie sie im wesentlichen in Tabelle II der Beschreibung angegeben sind, aufweist.

3. Verfahren nach Anspruch 1, worin der Zeolith ein Röntgenbeugungsmuster einschließlich der Werte, wie sie im wesentlichen in Tabelle III der Beschreibung angegeben sind, aufweist.

4. Verfahren nach Anspruch 1, worin der Zeolith ein Röntgenbeugungsmuster einschließlich der Werte, wie sie im wesentlichen in Tabelle IV der Beschreibung angegeben sind, aufweist.

5. Verfahren nach Anspruch 1, worin der Zeolith eine Zusammensetzung aufweist, die das Molverhältnis
X₂O₃:(n)YO₂
umfaßt, worin n mindestens etwa 10 ist, X ein dreiwertiges Element ist und Y ein vierwertiges Element ist.

6. Verfahren nach Anspruch 6, worin X Aluminium und Y Silicium umfaßt.

7. Verfahren nach Anspruch 1, worin der Zeolith Gleichgewichts-Adsorptionskapazitäten von mehr als 4,5 Gew.% für Cyclohexandampf und mehr als 10 Gew.-% für n-Hexandampf aufweist.

8. Verfahren nach Anspruch 1, worin das Alkylierungsmittel mindestens eine ungesättigte aliphatische Gruppe mit 2 bis 5 Kohlenstoffatomen umfaßt.

9. Verfahren nach Anspruch 8, worin das Alkylierungsmittel Propylen und die aromatische Verbindung Benzol und/oder Cumol ist.

10. Verfahren nach Anspruch 8, worin das Alkylierungsmittel Ethylen und die aromatische Verbindung Benzol ist.

11. Verfahren nach Anspruch 1, worin das Alkylierungsmittel wenigstens eine aliphatische Kette mit wenigstens 6 Kohlenstoffatomen umfaßt.

12. Verfahren nach Anspruch 11, worin die aromatische Verbindung ein Kohlenwasserstoff oder eine Phenolverbindung ist.

13. Verfahren nach Anspruch 1, worin die Alkylierungsbedingungen eine Temperatur von 0°C bis 500°C, einen Druck von 20 bis 25350 kPa (0,2 bis 250 atm), eine stündliche Gewichts-Raum-Geschwindigkeit von 0,1 bis 500 und ein Molverhältnis der alkylierbaren aromatischen Verbindung zum Alkylierungsmittel von 0,1:1 bis 50:1 einschließen.

14. Verfahren nach Anspruch 1, worin die Alkylierungsbedingungen eine Temperatur von 10°C bis 350°C, einen Druck von 100 bis 2550 kPa (1 bis 25 atm), eine stündliche Gewichts-Raum-Geschwindigkeit von 0,5 bis 100 und ein Molverhältnis der alkylierbaren aromatischen Verbindung zum Alkylierungsmittel von 0,5:1 bis 10:1 einschließen.

## Revendications

1. Un procédé d'alkylation d'un composé aromatique comprenant la mise en contact du composé aromatique avec au moins un agent alkylant en présence d'un catalyseur comprenant une zéolite cristalline poreuse synthétique présentant un spectre de diffraction aux rayons-X incluant des valeurs sensiblement telles que décrites dans le tableau I de la description.

2. Le procédé selon la revendication 1 dans lequel la zéolite présente un spectre de diffraction aux rayons-X incluant des valeurs sensiblement telles que décrites dans le tableau II de la description.

3. Le procédé selon la revendication 1 dans lequel la zéolite présente un spectre de diffraction aux rayons-X incluant des valeurs sensiblement telles que décrites dans le tableau III de la description.

4. Le procédé selon la revendication 1 dans lequel la zéolite présente un spectre de diffraction aux rayons-X incluant des valeurs sensiblement telles que décrites dans le tableau IV de la description.

5. Le procédé selon la revendication 1 dans lequel la zéolite présente une composition comprenant la relation molaire X₂O₃/(n)YO₂,
dans laquelle n est au moins environ égal à 10, X est un élément trivalent et Y est un élément tetravalent.

6. Le procédé selon la revendication 5 dans lequel X comprend de l'aluminium et Y comprend du silicium.

7. Le procédé selon la revendication 1 dans lequel la zéolite présente des capacités d'adsorption à l'équilibre supérieures à 4,5% en poids pour la vapeur de cyclohexane et supérieures à 10% en poids pour la vapeur de n-hexane.

8. Le procédé selon la revendication 1 caractérisé en ce que l'agent d'alkylation comprend au moins un groupe aliphatique insaturé comportant de 2 à 5 atomes de carbone.

9. Le procédé selon la revendication 8 dans lequel l'agent alkylant est du propylène et le composé aromatique est du benzène et/ou du cumène.

10. Le procédé selon la revendication 8 dans lequel l'agent alkylant est de l'éthylène et le composé aromatique est du benzène.

11. Le procédé selon la revendication 1 dans lequel l'agent alkylant comprend au moins une chaîne aliphatique comportant au moins 6 atomes de carbone.

12. Le procédé selon la revendication 11 dans lequel le composé aromatique est un hydrocarbure ou un composé phénolique.

13. Le procédé selon la revendication 1 dans lequel les conditions d'alkylation incluent une température de 0 à 500°C, une pression de 20 à 25350 kPa (0,2 à 250 atmosphères), une vitesse spatiale horaire pondérale de 0,1 à 500 et un rapport molaire composé aromatique susceptible d'être alkylé/agent alkylant de 0,1/1 à 50/1.

14. Le procédé selon la revendication 1 dans lequel les conditions d'alkylation incluent une température de 10 à 350°C, une pression de 100 à 2550 kPa (1 à 25 atmosphères), une vitesse spatiale horaire pondérale de 0,5 à 100 et un rapport molaire composé aromatique susceptible d'être alkylé/agent d'alkylant de 0,5/1 à 10/1.
